# EUROPEAN PATENT APPLICATION

(11) **EP 3 778 613 A1**
(43) Date of publication of application: **17.02.2021**
(21) Application number: 19382708.6
(22) Date of filing: 12.08.2019
(51) Int. Cl.: C07F 9/6593, A61K 49/12

(54) **THIRD GENERATION DENDRIMER, PROCESS FOR PRODUCTION THEREOF AND USE THEREOF**

(71) Applicant: Consejo Superior de Investigaciones Científicas (CSIC), 28006 Madrid (ES)
(72) Inventor: VIDAL GANCEDO, José, 08193 Cerdanyola del Vallès (Barcelona) (ES); LLOVERAS MONSERRAT, Vega, 08193 Cerdanyola del Vallès (Barcelona) (ES)
(74) Representative: Pons

(57) **Abstract**

The present invention discloses a third generation polyphosphorhydrazone (PPH) dendrimer peripherally functionalized with PROXYL radical units, by using a tyrosine amino acid (Tyr) as linker. The present invention also relates to the process of obtainment of said dendrimer and the use thereof as a magnetic resonance imaging (MRI) contrast agent.

## Description

The invention relates to polyphosphorhydrazone (PPH) based Tyr-PROXYL radical dendrimers of third generation and use thereof as a magnetic resonance imaging (MRI) contrast agents. Therefore, the present invention belongs to the medicine field.

### STATE OF ART

Gadolinium (Gd) based contrast agents (CA) are the most widely used CA in magnetic resonance imaging (MRI). They are categorized as mainly *T*₁ CA and present high relaxivities due to the high spin of paramagnetic Gd (III) ion (spin 7/2). These CA have historically been considered as safe. However, for more than a decade, a linear relationship between their administration and the development of nephrogenic systemic fibrosis has been recognized in patients with severe renal impairment. Recent reports have emerged regarding the accumulation of residual toxic Gd ions in the brain, bones, skin, liver and kidneys of patients with normal renal function and intact blood-brain barrier, following the administration of these Gd-based CA. Since the use of CA in MRI is of vital importance to gain lifesaving clinical information, it is critical to find alternative imaging probes that own the same or even better paramagnetic properties of current Gd CA. Stable organic free radicals, such as nitroxides, have been investigated as T₁ CA for MRI. They were shown minimal toxicity for in vivo use, in comparison with Gd-based CA. Since in the case of nitroxides, MRI contrast is achieved via paramagnetic water relaxation, they could, in principle, be immediately translated to clinical applications. Despite all of these, the widespread application of nitroxides as MRI CA has been rather limited, due to their inherent low paramagnetic relaxivity and rapid bioreduction. One strategy to achieve higher molecular relaxivity is through the incorporation of many nitroxyl units to a polymer or a hyperbranched polymer, where the relatively low relaxivity per nitroxide is multiplied by the number of bounded nitroxides. Moreover, a protective shield effect could be provided to the radicals, by restricting the fast access of reducing agents.

When discussing about hyperbranched polymers it is inevitable to think about dendrimers. Despite their architectural resemblance at first sight, dendrimers differ by the strict control over their structure, making them nearly perfect monodisperse macromolecules. Their tunable size and multifunctionality make them excellent candidates for theranostic nanomedicine and dual modality bio-imaging. Indeed, dendrimers have attracted much interest for the development of new MRI CAs, most of them based on the incorporation of Gd chelates in their periphery. Only few reports describe their functionalization with organic radicals for MRI CA application, being most of these works devoted to study their electronic properties. In those few reports, PAMAM and PPI dendrimers were functionalized with 2,2,5,5-tetramethylpyrrolidin-1-oxyl (PROXYL) radical units (Winalski, C. S. et al. Magn. Reson. Med. 2002, 48, 965) or nitronyl nitroxides (NIT) radicals (Francese, G. et al. Magn. Reson. Chem. 2003, 41, 81) resulting in a high paramagnetic system with a high molecular relaxivity due to the large number of radical units. However, for higher generations (Gn), the relaxivity per unit of radical decreases. It can be due to aggregation issues promoted by lower solubility of larger Gn, what makes radical units inaccessible by water molecules. Indeed, PAMAM and PPI dendrimers have tertiary amines that are incompletely protonated in physiologic pH.

Aware of the potential of radical dendrimers as an entirely organic metal-free MRI CA alternative, the present invention describes for the first time the synthesis and structural characterization of new PPH-based dendrimers of the third generation and their use as highly efficient MRI contras agents.

### DESCRIPTION OF THE INVENTION

The present invention discloses a third generation polyphosphorhydrazone (PPH) dendrimer peripherally functionalized with PROXYL radical units, by using a tyrosine amino acid (Tyr) as linker. Tyr was coupled to the dendrimer via the phenolic hydroxyl group, providing an available amino group for radical coupling and a negative charge to confer high solubility.

Then, a first aspect of the present invention relates to a compound of formula (I): 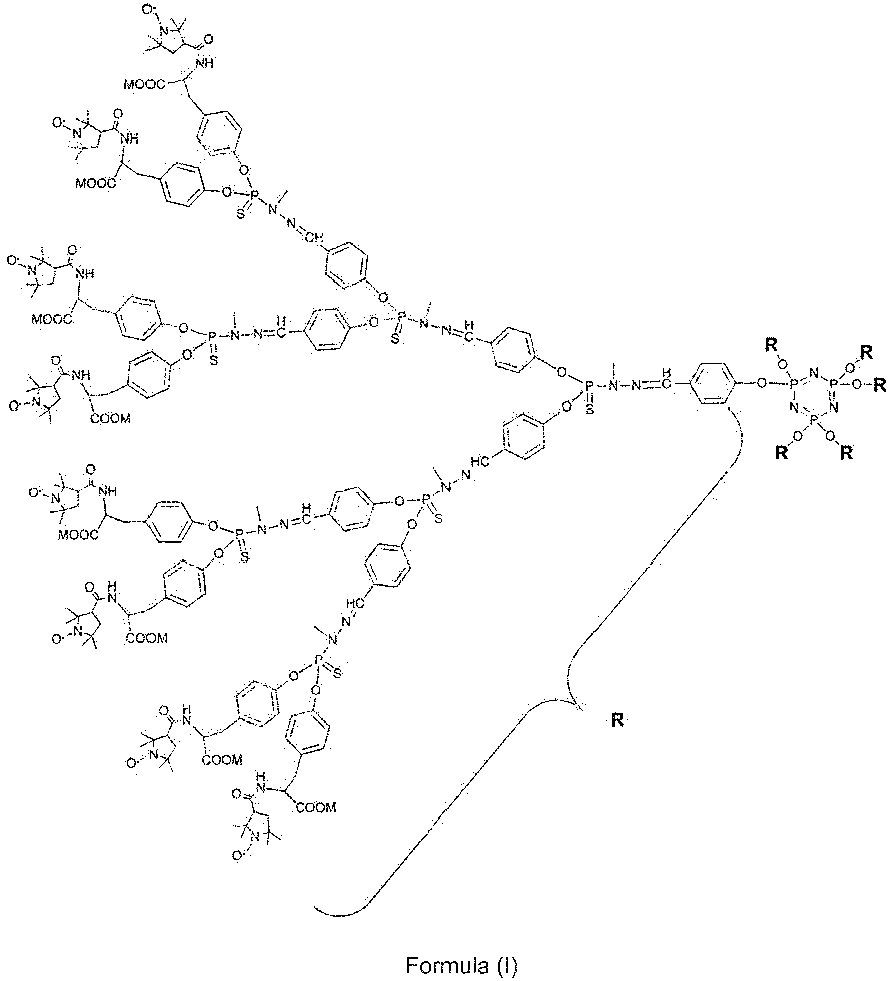 its tautomers and/or solvates, wherein M is a cation selected from Li⁺, Na⁺ or K⁺, more preferably, M is Li⁺.

The compound of formula (I) is a dendrimer of third generation functionalized with PROXYL radical units. It is also referred in the present invention as G3-Tyr-PROXYL dendrimer.

The compound of formula (I) can also be represented by the next simplified formula:

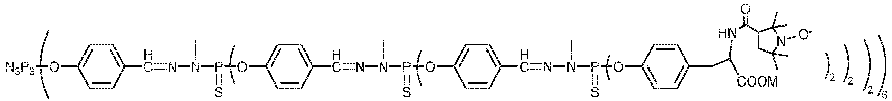

The term "dendrimer" refers to repetitively branched molecules. The dendrimer may be assembled from a multifunctional core, which is extended outward by a series of reactions, step by step, each growing step referred to as a "generation". A dendrimer is typically symmetric around the core where the number of branches increase with the generation and often adopts a spherical three-dimensional morphology. Dendrimers differ from other polymers by the strict control over their structure, making them nearly perfect monodisperse macromolecules.

Unless otherwise stated, the compounds used in the invention are intended to include compounds that differ only in the presence of one or more isotopically enriched atoms. For example, compounds having the present structures except for the substitution of a hydrogen atom for a deuterium atom or a tritium atom, or the substitution of a carbon atom for a carbon atom enriched in ¹³C or ¹⁴C or a nitrogen atom enriched in ¹⁵N fall within the scope of this invention.

The compounds used in the invention may be in non-crystalline form, either as free compounds or as solvates (e.g.: hydrates), and it is understood that both forms fall within the scope of the present invention. Solvation methods are generally known in the state of the art. Suitable solvates are pharmaceutically acceptable solvates. In a particular embodiment, the solvate is a hydrate.

"Tautomers" are understood to be the two isomers that differ only in the position of a functional group because between the two forms there is a chemical balance in which a migration of a group or atom occurs.

A second aspect of the invention relates to a pharmaceutical composition comprising the compound of formula (I) described in the first aspect of the invention. Preferably, the pharmaceutical composition comprises a pharmaceutically acceptable excipient, carrier or adjuvant.

The term "excipients, adjuvants and/or carriers" relates to molecular entities or substances through which the active ingredient is administered. Such pharmaceutical excipients, adjuvants or carriers can be sterile liquids, such as water and oils and also can be disintegrating agents, humectants or dilutes.

The composition is preferably configured to be administered to the patient in the form of an injectable composition. The method of administering the composition is preferably parenterally. Suitable parenteral administration routes include intravascular administration (e.g. intravenous bolus injection, intravenous infusion, intra-arterial bolus injection, intraarterial infusion and catheter instillation into the vasculature); peri- and intra-tissue injection; subcutaneous injection or deposition including subcutaneous infusion (such as by osmotic pumps); and direct application to tissue, for example by a catheter or other placement device (e.g. a suppository or an implant comprising a porous, non-porous, a gelatinous material, or a fiber).

In a preferred embodiment, the composition is formulated in accordance with routine procedures as a pharmaceutical composition adapted for intravenous administration to a subject. Typically, compositions for intravenous administration are solutions in sterile isotonic aqueous buffer. Where necessary, the composition may also include a solubilizing agent and a local anaesthetic such as lignocaine to ease pain at the site of the injection. Generally, the ingredients will be supplied either separately or mixed together in unit dosage form, for example, as a dry lyophilized powder or water free concentrate. The composition may be stored in a hermetically sealed container such as an ampule or sachet indicating the quantity of active agent in activity units. Where the composition is administered by infusion, it can be dispensed with an infusion bottle containing sterile pharmaceutical grade "water for injection" or saline. Where the composition is to be administered by injection, an ampule of sterile water for injection or saline may be provided so that the ingredients may be mixed prior to administration.

A third aspect of the present invention relates to compounds of formula (II): 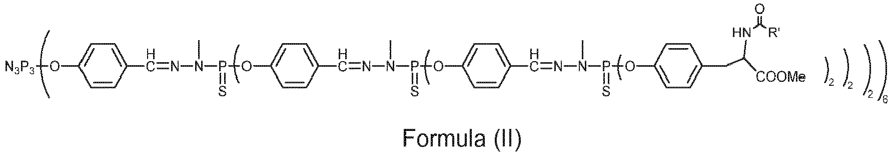 wherein Me is methyl and R' is: 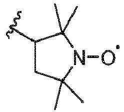 or other organic radicals, its tautomers and/or solvates.

The compound of formula (I) can be obtained from the compound of formula (II) as explained in the next aspect of the present invention.

A further aspect of the present invention relates to a process of obtainment of compound of formula (I) from the compound G3-Cl: 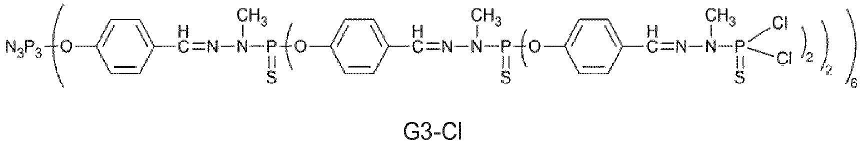 wherein the process comprises at least the following steps:
a) Terminal chlorine groups attached to the phosphorus atoms of the compound G3-Cl are replaced by units of *N*-Boc-protected tyrosine methyl ester (Boc-Tyr-OMe), via phenolic groups, affording G3-Boc-Tyr-OMe dendrimer: 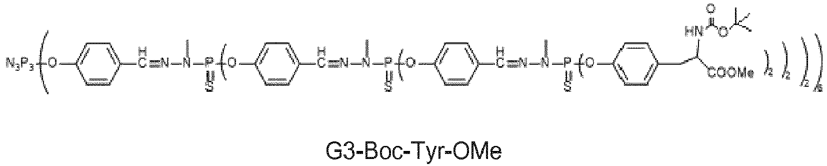
b) Subsequently, the amine groups of tyrosine were deprotected with trifluoroacetic acid and coupled with 3-carboxy-PROXYL radicals using HATU as coupling reagent and DIPEA as base to obtain the G3-(Tyr-OMe)-PROXYL dendrimer: 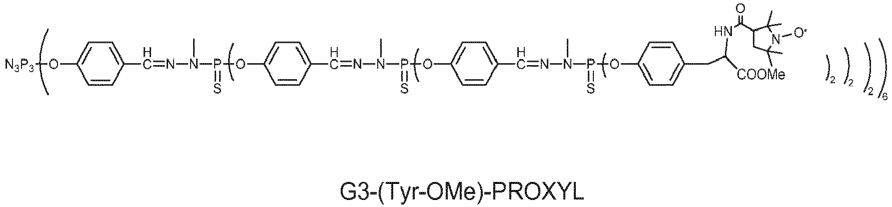
c) The methyl esters were hydrolyzed with LiOH, KOH or NaOH, resulting in the water soluble compound of formula (I) (G3-Tyr-PROXYL dendrimer) containing 48 terminal Tyr-PROXYL:

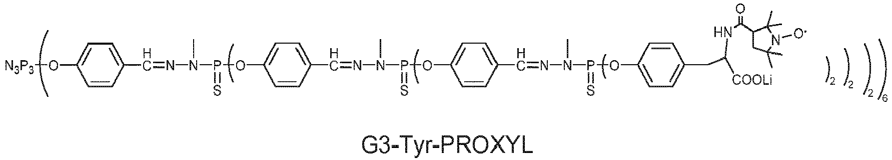

In a preferred embodiment, step a) is carried out in basic conditions using THF as a solvent.

In a preferred embodiment, the dendrimers obtained in step a) are purified by ultrafiltration. By this technique, the dendrimer in step a) is obtained with high purity, since it is possible to eliminate all the impurities with a size smaller than the dendrimer obtained in step a).

In a preferred embodiment, step b) is carried out in in CH₂Cl₂.

In a preferred embodiment, the dendrimers obtained in step b) are purified by column chromatography and ultrafiltration. By this technique, the dendrimer in step b) is obtained with high purity (>95%, generally between 98-100%, measured by Size Exclusion Chromatography, SEC).

In a preferred embodiment, step c) is carried out in THF/H₂O.

In a preferred embodiment, in step c), the methyl esters were hydrolyzed with LiOH.

Another aspect of the invention refers to the use of the compound of formula (I) as a magnetic resonance imaging (MRI) contrast agent.

The compound of formula (I) (dendrimer of third generation) presents excellent properties to be used as a contrast agent. It shows an impressive four times higher T₁ relaxivity in comparison to Gd-DTPA used in clinics, without having the risks associated with the unwanted accumulation of Gd in the body. Besides, said dendrimer presents higher relaxivity per molecule than dendrimers of lower generations. In particular, it has a relaxivity per molecule that is more than 2 times higher than the relaxivity of the second-generation dendrimer. And what is even more surprising is that its relaxivity per radical is a 16% higher than the relaxivity per radical of any of G0-G2 dendrimers (see examples of the invention).

The compounds of formula (I) offer to the pendant radicals a higher stability against reduction and a higher paramagnetic relaxivity due to the presence of a large number of radicals in the same molecule.

In addition, the compound of formula (I) do not show any cytotoxicity in vitro, in the tested concentration range (from 0.016 to 2mM per radical), therefore it is biocompatible useful for clinical applications.

For use as a contrast agent, the dendrimer is ordinarily injected as a solution in a nontoxic injectable pharmacologically acceptable sterile aqueous vehicle, e. g., distilled water physiological saline solution. The aqueous vehicle can also contain other ingredients conventionally present in solutions, e. g., local anesthetic, anti-inflammatory agent, e. g., cortisone, antibiotic, stabilizing agent, suspending agent, etc.

The dosage to be administered, and the mode of administration will depend on a variety of factors including age, weight, sex, condition of the patient and genetic factors, and will ultimately be decided by medical personnel subsequent to experimental determinations of varying dosage followed by imaging as described herein. A dosage required for diagnostic sensitivity or therapeutic efficacy could range from about 0.01 and 0.03 mmol/kg of host body mass.

Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following examples and drawings are provided by way of illustration and are not intended to be limiting of the present invention.

### BRIEF DESCRIPTION OF THE DRAWINGS

FIG. 1. Structures of the Gn-Tyr-PROXYL dendrimers with n= 0-3
FIG. 2. a) X-band EPR spectra (CH₂Cl₂, 300K) of 0.21 mM solutions of 3-carboxy-PROXYL and Gn-(Tyr-OMe)-PROXYL (n= 0-3) dendrimers. b) Double integral of the EPR spectra *vs* number of PROXYL units.
FIG. 3. a) X-band EPR spectra (100 mM phosphate buffer pH 7.4, 300K) of 0.21 mM solutions of Gn-Tyr-PROXYL (n= 0-3) dendrimers. b) Double integral of the EPR spectra of Gn-Tyr-PROXYL dendrimers (0.21 mM, 100 mM PB pH 7.4, 180 K) *vs* number of PROXYL units.
FIG. 4. *a*) First 20 minutes of reduction of 2 mM of PROXYL with 20 mM of ascorbate, of 3-carboxy-PROXYL and Gn-Tyr-PROXYL dendrimers in 100 mM phosphate buffer (pH 7.4) at 300 K, measured by EPR (the lines are guide to the eyes). *b*) Half-life times calculated from the first stage of reduction (30 min - 1 h of reduction).
FIG. 5. *a*) Longitudinal (*r*₁) and transverse (*r*₂) relaxivity per molecule of Gn-Tyr-PROXYL dendrimers determined at 7T in 100 mM phosphate buffer pH 7.4, 300K. *b*) Relaxivity per radical of Gn-Tyr-PROXYL dendrimers.
FIG. 6. *In vitro* viability assays conducted with a fetus normal lung tissue cell line (MRC-5) incubated with Gn-Tyr-PROXYL dendrimers (n=0-3) in a concentration of 2mM per radical for 24 and 48 h.
FIG. 7. *In vivo* T1-weighted MRI images of a mouse body without CA (left) and with 0.00625 mmol/Kg of G3-Tyr-PROXYL radical dendrimers (right).

### EXAMPLES

The inventors have prepared the dendrimer of third generation (G3-Tyr-PROXYL) of the present invention that can be used as a contrast agent. Additionally, they have prepared the dendrimers of lower generations (G0 to G2) in order to make a comparative study between them.

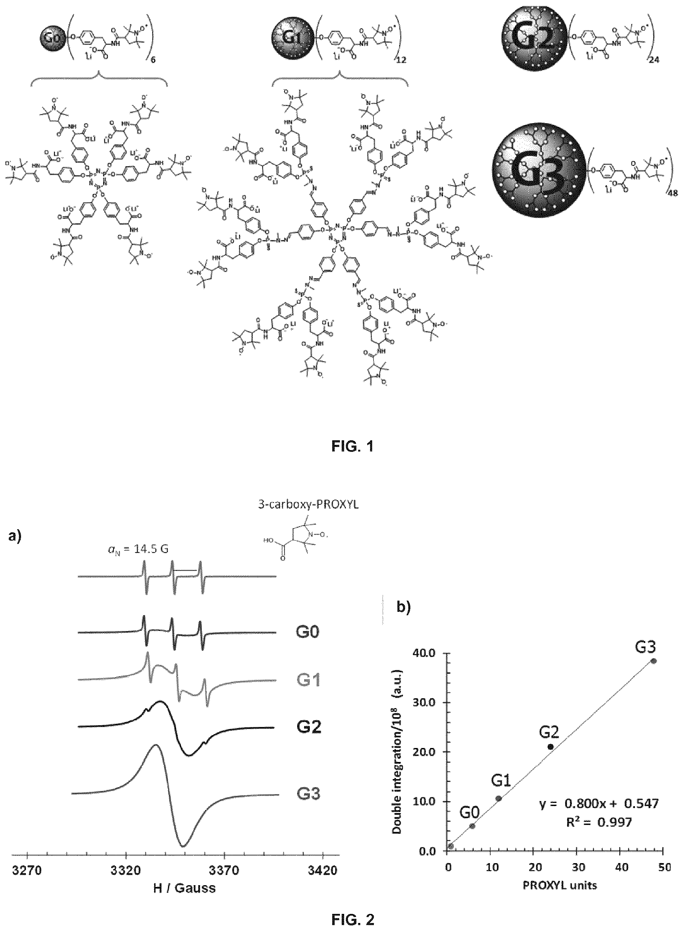

The dendrimers are referred as Gn-Tyr-PROXYL, wherein n (n=0, 1, 2, 3) indicates the generation of the dendrimer. In Fig. 1, the structures of the Gn-Tyr-PROXYL dendrimers with n= 0-3 are represented.

Their magnetic properties were investigated by Electronic Paramagnetic Resonance Spectroscopy (EPR), followed by the evaluation of their relaxivity, resistance against chemical reduction and cytotoxicity.

Also, in vivo studies have been carried out with mice. Anesthesia was performed with isoflurane (B.Braun, Melsungen, Germany) at 0.5-1.5% in O₂, maintaining the respiratory frequency between 40-60 breaths/min. Respiration rate was constantly monitored (SA Instruments, Inc., New York, USA). Before immobilization in the animal holder, each mouse was cannulated in the tail vein using a home-built multi-delivery polyethylene tubing system loaded with saline solution (NaCl 0.9%, B.Braun) and G3-Tyr-PROXYL radical dendrimers as contrast agent. The contrast agent was injected into the mice as a bolus (100 µL, about 0.00625 mmol/kg) during Dynamic Contrast Enhanced (DCE)-T1 MRI studies.

### 1. Materials and Methods

### 1.1. Materials

Hexachlorocyclotriphosphazene, 4-hydroxybenzaldehyde, thiophosphoryl chloride, methylhydrazine, N-(tert-Butoxycarbonyl)-L-tyrosine methyl ester (Boc-Tyr-OMe), Cs₂CO₃, 3-carboxy-PROXYL, (1-[Bis(dimethylamino)methylene]-1H-1,2,3-triazolo[4,5-b]pyridinium 3-oxid hexafluorophosphate (HATU), N,N-diisopropylethylamine (DIPEA) and LiOH.H₂O were obtained from Sigma-Aldrich. Trifluoracetic acid (TFA) was obtained from EMD Millipore. CH₂Cl₂ and CHCl₃ were distillated from CaH₂. Tetrahydrofuran (THF) was distillated from Na/benzophenone. Ultrafiltration was performed on solvent-resistant stirred cells from EMD Millipore with regenerated cellulose membranes (1, 3, 5, 10 and 30 kDa). Ultrapure water (Milli-Q, EMD Millipore) and HPLC grade acetone was used for ultrafiltration. All aqueous solutions used in EPR and MRI experiments were prepared with ultrapure water (Milli-Q, EMD Millipore).

### 1.2. Methods

**Size exclusion chromatography (SEC)** analysis was carried out using an Agilent 1260 infinity II liquid chromatography system apparatus equipped with a diode array detector under the following conditions. For Gn-Boc-Tyr dendrimers, a PSS SDV pre-column (3 µm, 8 x 50 mm) and a PSS SDV analytical column (3 µm, 1000 Å, 8 × 300 mm) with a diode array detector were used. CHCl₃ was used as eluent at a flow rate of 0.5 mL/min at 35 °C. Dendrimers were dissolved in the eluent to reach a final concentration of 2 mg/mL for G0, 1.5 mg/mL for G1 and 1 mg/mL for G2 and G3 dendrimer and filtered through 0.2 µm PTFE filter before injection. For Gn-Tyr-PROXYL dendrimers, a PSS Suprema pre-column (10 µm, 8 x 50 mm) and a PSS Suprema analytical column (10 µm, 100 Å, 8 × 300 mm) with a diode array detector were used. LiCl 0.25 mM in water was used as eluent at a flow rate of 0.35 mL/min at 35 °C. Dendrimers were dissolved in the eluent to reach a final concentration of 2 mg/mL for G0, 1.5 mg/mL for G1 and 1 mg/mL for G2 and G3 dendrimer and filtered through 0.2 µm nylon filter before injection.

**Electronic Paramagnetic Resonance Spectroscopy (EPR)** spectra were obtained with an X-Band (9.4 GHz) Bruker ELEXSYS 500 spectrometer equipped with a TE102 microwave cavity, a Bruker variable temperature unit, a field frequency lock system Bruker ER 033 M and equipped with a NMR Gaussmeter Bruker ER 035 M. The modulation amplitude was kept well below the line width, and the microwave power was well below saturation. All samples were previously degassed with Ar. Experiments in aqueous phase were carried out with a quartz flat cell.

**Kinetics of reduction of PROXYL with ascorbate.** All the solutions were freshly prepared in 100 mM phosphate buffer, pH 7.4. 300 µL each of 3-carboxy-PROXYL (2.5 mM) or Gn-Tyr-PROXYL dendrimers (2.5mM per PROXYL) and 240 µL of 100 mM phosphate buffer solution were mixed in a 1.5 mL Eppendorf® microtube. This solution was degassed using argon flux during 20 s. Next, 60 µL of sodium ascorbate (5 mM) was added. This solution was mixed for 6 seconds and transferred immediately to a quartz flat cell for the EPR spectra acquisition.

**Matrix-Assisted Laser Desorption/lonization- time-of-flight mass spectrometer (MALDI-TOF MS)** Bruker Daltonic system was used to acquire the mass spectra of G0-Boc-Tyr-OMe and GO-(Tyr-OMe)-PROXYL dendrimers. Dithranol was used as matrix. **Nuclear Magnetic Resonance Spectroscopy (NMR)** experiments were recorded at Bruker spectrometers Avance DRX-250, DXP-360 and Advance III-400 using chloroform-d (CDCl₃) as solvent. ¹H and ¹³C NMR chemical shifts (δ) are reported in ppm relative to residual CHCl₃ at 7.26 and 77.16 ppm respectively. ³¹P{¹H} NMR chemical shift are reported in ppm relative to 85% phosphoric acid (5 = 0 ppm), used as an external standard.

**Magnetic Resonance Imaging (MRI)** experiments were carried out in a BioSpec 70/30 Bruker system using a 7.0 T horizontal-bore superconducting magnet equipped with actively shielded gradients (B-GA12 gradient coil inserted into a B-GA20S gradient system). A quadrature 72 mm inner diameter volume coil was used for *in vitro* studies.

**Relaxometric measurements.** Longitudinal (*r*₁) and transverse (*r*₂) relaxivities were determined per concentration of PROXYL units. Six different concentrations of PROXYL radicals (0.25 - 10 mM) in 30 mM phosphate buffer (pH 7.4) were prepared. Relaxivity measurements were obtained at 37 °C. The software used for the calculations of *T*₁ and *T*₂ relaxations was Paravision 6.0 (Bruker Software). ***r*₁ relaxivity.** Series of axial *T*₁-weighted (T1W) images were acquired for each concentration of PROXYL to obtain *T*₁ maps based on a magnetization saturation experiment and the following parameters: repetition time (TR) = 70-6000 ms, echo time (TE) = 9.2 ms, field of view (FOV) = 2.5 x 2.5 cm, averages (Av) = 1, acquisition matrix (Mtx) = 128 x 128. The *T*₁ values were calculated from the mean signal in the region of interest (ROI) for each repetition time, adjusted to the equation: *S* = *S₀* [1 - (-TR/*T*₁)]. ***r*₂ relaxivity.** *T*₂ maps were calculated from multi spin echo images with a TR of 2 s. 25 echo images were acquired with a TE of 8.02 ms, which was the interval time between echo images acquisitions, too. *T*₂-map parameters were as follows: 1 axial slice of 2 mm thickness; TR = 3200 ms; TE =8.02 ms (25 echo times with interval of 8.02 ms in between), 1 average. Field of view (FOV): 35 x 35 mm; Mtx: 256 x 256. The *T*₂ values were calculated from mean signal in the ROI for each echo image, adjusted according to the equation: *S* = *S₀* (-TE/*T*₂)].

**Dynamic Light Scattering (DLS)** experiments were carried out on a Nano-S Zetasizer (Malvern Instrument Ltd) with back scattering detector (173°, 633 nm laser wavelenght) to measure hydrodynamic size (diameter) in batch mode at 25 °C. Dendrimers samples were prepared at a concentration of 1 mg/mL in 30 mM phosphate buffer pH 7.4, with and without the addition of 9% NaCl. The samples were filtered through 0.2 µm PTFE filter before analysis. A minimum of 5 measurements per sample were made. Hydrodynamic size was reported as the volume-weighted average.

### 2. Synthesis and characterization

**Synthesis.** The synthetic procedure for Gn-Tyr-PROXYL (with n=0,1,2,3) dendrimers is shown in Scheme 1.

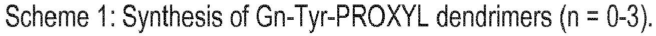

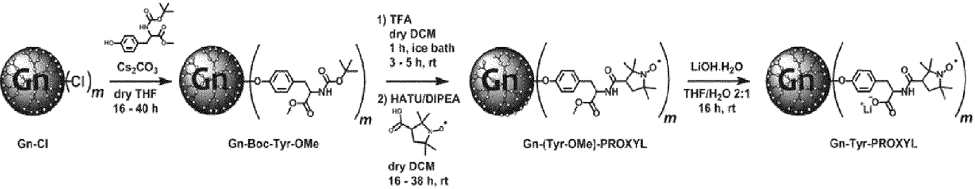

The starting point of the synthesis was the hexachlorocyclotriphosphazene (Gn-Cl, n=0) and the P(S)Cl₂ terminated PPH dendrimers (Gn-Cl, n=1,2,3), containing a hexafunctional N₃P₃ core. (Gn-Cl, n=1,2,3) dendrimers were obtained and characterized as previously reported (Launay, N. et al. J. Am. Chem. Soc. 1995, 117, 3282; Launay, N. et al. Journal of Organometallic Chemistry 1997, 529, 51). Terminal chlorine groups attached to the phosphorus atoms were replaced by units of N-Boc-protected tyrosine methyl ester (Boc-Tyr-OMe), via phenolic groups, in basic conditions using THF as a solvent, to afford Gn-Boc-Tyr-OMe dendrimers. The full functionalization of the dendrimers with Boc-Tyr-OMe groups was verified by ¹H NMR spectroscopy and their purity monitored by size exclusion chromatography (SEC). Subsequently, the amine groups of tyrosine were deprotected with trifluoroacetic acid and coupled with 3-carboxy-PROXYL radicals using HATU as coupling reagent and DIPEA as base in CH₂Cl₂ to obtain Gn-(Tyr-OMe)-PROXYL dendrimers. Finally, the methyl esters were hydrolyzed with LiOH in THF:H₂O (2:1), resulting in the water soluble Gn-Tyr-PROXYL (with n= 0-3) dendrimers, containing theoretically 6, 12, 24 and 48 terminal Tyr-PROXYL groups and molecular weight ranging from 2261 to 26013 g·mol⁻¹. All the steps of synthesis involving the presence of PROXYL radicals were carried out in the dark room (dark conditions). Gn-Boc-Tyr-OMe dendrimers were purified by precipitation with THF/n-pentane. For G2 and G3, it was followed by column chromatography and ultrafiltration, respectively. Ultrafiltration was also used to purify Gn-(Tyr-OMe)-PROXYL and Gn-Tyr-PROXYL dendrimers, with the exception of G1-(Tyr-OMe)-PROXYL dendrimers that were purified by column chromatography. Due to the presence of paramagnetic PROXYL units, the full functionalization of radical dendrimers was verified by EPR and their purity monitored by SEC.

Next, the synthesis of the dendrimers Gn-Tyr-PROXYL, including reaction intermediates, are explained in more detail:
**GO-Boc-Tyr-OMe.** In a Shlenk round-bottomed flask equipped with a stir bar under argon atmosphere, hexachlorocyclotriphosphazene (200 mg, 575 µmol, 1 eq), Cs₂CO₃ (3.0 g, 9.2 mmol, 16 eq) and Boc-Tyr-OMe (1.27 g, 4.32 mmol, 7.5 eq) were added and suspended in anhydrous THF (10 mL). The reaction mixture was let under vigorous stirring during 16 hours at room temperature. The reaction was followed by ³¹P NMR by the disappearance of the ³¹P signal at 20.1 ppm. Once the reaction was finished, the solid residue was separated from the organic phase by filtration and the organic phase was evaporated to dryness. The crude product was dissolved in a minimum volume of THF and precipitated several times with n-pentane, to afford GO-Boc-Tyr-OMe (1.0 g, 91%) as a pale yellow solid. ³¹P{¹H} NMR (100 MHz, CDCl₃) δ: 8.67; ¹H NMR (360 MHz, CDCl₃) δ: 7.05-6.72 (m, 24H), 5.15 (s, 6H), 4.44 (s, 6H), 3.60 (s, 18H), 3.05-2.80 (m, 12H), 1.32 (s, 54H); ¹³C NMR (90.5 MHz, CDCl₃) δ: 172.2, 155.1, 149.5, 132.8, 130.1, 120.8, 79.8, 54.4, 52.1, 37.4, 28.2. MALDI-TOF MS (dithranol, linear mode m/z): 1907. Calcd. for [M+H]⁺, C₉₀H₁₂₀N₉O₃₀P₃: 1902

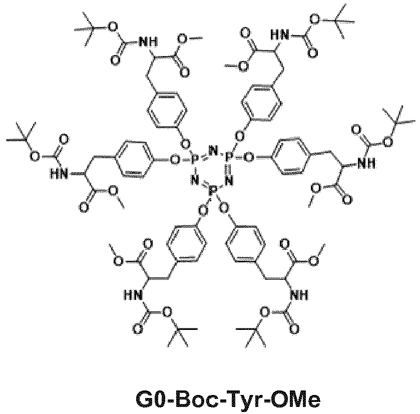

**GO-(Tyr-OMe)-PROXYL.** In a Shlenk round-bottomed flask equipped with a stir bar under argon atmosphere, GO-Boc-Tyr-OMe (500 mg, 263 µmol, 1 eq) was dissolved in anhydrous CH₂Cl₂ (5 mL). The solution was cooled in an ice bath and TFA (3.65 mL, 180 eq) was added dropwise during 20 min. The ice bath was removed, and the reaction mixture was stirred at room temperature for 5 h. Next, the solvent was evaporated to dryness and the crude was dissolved and evaporated several times with CH₃OH to remove TFA residue. Under dark conditions, the crude was transferred to a Shlenk round-bottomed flask equipped with a stir bar and dried under vacuum. In another Shlenk round-bottomed flask equipped with a stir bar and under argon atmosphere, 3-carboxy-PROXYL (382 mg, 2.05 mmol, 7.8 eq) and HATU (780 mg, 2.05 mmol, 7.8 eq) were dissolved in anhydrous CH₂Cl₂ (15 mL). Afterwards, DIPEA (2.75 mL, 15.78 mmol, 60 eq) was added. After being stirred for 20 minutes, this solution was transferred dropwise via cannula to the flask containing the crude. The reaction mixture was let to stir at room temperature during 24 hours. The solvent was evaporated and the crude product was dissolved in CH₂Cl₂ and washed with H₂O (4 x 50 mL). The organic phase was dried over MgSO₄ and concentrate under reduced pressure to give a crude product that was subjected to column chromatography using CH₂Cl₂:CH₃OH (10:1) as eluent. The concentrated was collected and the solvent evaporated to afford GO-(Tyr-OMe)-PROXYL (400 g, 66%) as a yellowish oil. MALDI-TOF MS (dithranol, linear mode m/z): 2311. Calcd. for [M+H]⁺, C₁₁₄H₁₅₆N₁₅O₃₀P₃: 2310

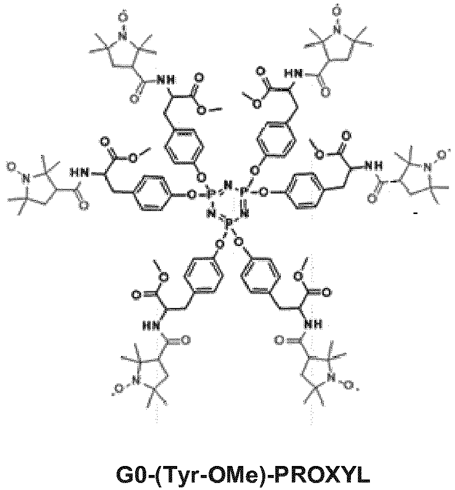

**GO-Tyr-PROXYL.** Under dark conditions, GO-(Tyr-OMe)-PROXYL (227 mg, 98.3 µmol, 1 eq) and LiOH·H₂O (347 mg, 8.27 mmol, 84 eq) were dissolved in THF/H₂O 2:1 solution (15 ml) in a round-bottomed flask equipped with a stir bar. The reaction mixture was let to stir at room temperature during 21 hours. Afterwards, the solvent was evaporated to dryness to give a crude product that was purified by ultrafiltration (regenerated cellulose membrane 1kDa, 7 x 40 mL water). The concentrated was collected and the water eliminated by freeze-drying to afford GO-Tyr-PROXYL (142 mg, 64%) as a pale yellow solid.

**G1-Boc-Tyr-OMe.** In a Shlenk round-bottomed flask equipped with a stir bar under argon atmosphere, G1-Cl (255 mg, 140 µmol, 1 eq), Cs₂CO₃ (2.18 g, 6.70 mmol, 48 eq) and Boc-Tyr-OMe (659 mg, 2.23 mmol, 16 eq) were suspended in anhydrous THF (16 mL). The reaction mixture was let under vigorous stirring during 16 hours at room temperature. The solid residue was separated from the organic phase by filtration. The organic phase was collected and centrifuged to remove the remaining solid residue. Next, the solvent was evaporated and the resulting crude product was dissolved in a minimum volume of THF and precipitated several times with n-pentane to afford G1-Boc-Tyr-OMe (650 mg, 94 %) as a pale yellow solid. ³¹P{¹H} NMR (100 MHz, CDCl₃) δ: 63.23, 8.89; ¹H NMR (360 MHz, CDCl₃) δ: 7.66-7.52 (m, 18H), 7.12-6.92 (m, 60H), 5.10 (d, *J* = 8.3 Hz, 12H, ), 4.56-4.42 (m, 12H), 3.61 (s, 36H), 3.22 (d, *J* = 10.1 Hz, 18H), 3.07-2.86 (m, 24H), 1.36 (s, 108H); ¹³C NMR (90.5 MHz, CDCl₃) δ: 172.3, 155.2, 151.3, 138.8, 133.5, 131.0, 130.5, 128.4, 121.4, 80.1, 54.5, 52.3, 37.8, 33.1, 28.4.

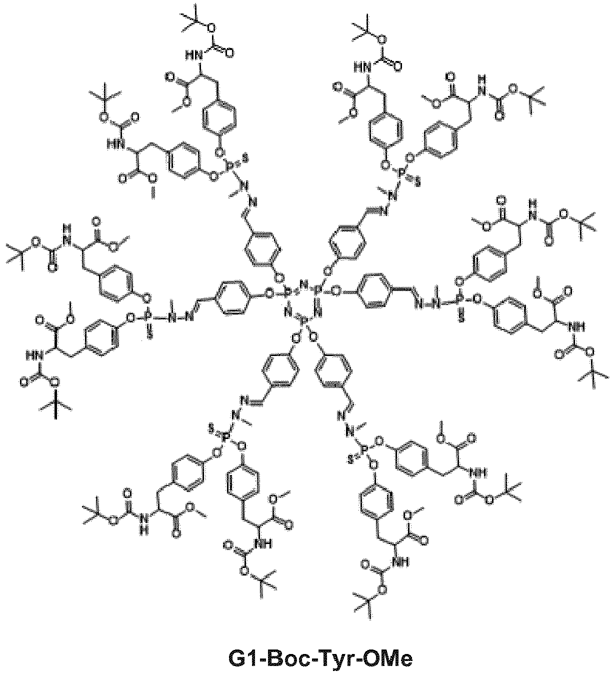

**G1-(Tyr-OMe)-PROXYL.** In a Shlenk round-bottomed flask equipped with a stir bar under argon atmosphere, G1-Boc-Tyr-OMe (500 mg, 101 µmol, 1 eq) was dissolved in anhydrous CH₂Cl₂ (6 mL). The solution was cooled in an ice bath and TFA (2.81 mL, 360 eq) was added dropwise during 20 min. The ice bath was removed, and the reaction mixture was stirred at room temperature for 5 h. Then, the solvent was evaporated to dryness and the crude was dissolved and evaporated several times with CH₃OH to remove TFA residue. Under dark conditions, the crude was transferred to a Shlenk round-bottomed flask equipped with a stir bar and dried under vacuum. In another Shlenk round-bottomed flask equipped with a stir bar and under argon atmosphere, 3-carboxy-PROXYL (294 mg, 1.58 mmol, 15.6 eq) and HATU (780 mg, 2.05 mmol, 15.6 eq) were dissolved in anhydrous CH₂Cl₂ (15 mL). Afterwards, DIPEA (2.12 mL, 12.1 mmol, 120 eq) was added. After being stirred for 20 minutes, this solution was transferred dropwise via cannula to the flask containing the crude. The reaction mixture was let to stir at room temperature during 24 hours. The solvent was evaporated and the crude product was dissolved in CH₂Cl₂ and washed with H₂O (4 x 50 mL). The organic phase was dried over MgSO₄ and concentrate under reduced pressure to give a crude product that was purified by ultrafiltration (regenerated cellulose membrane 3kDa, 7 x 40 mL acetone). The concentrated was collected and the solvent evaporated to afford G1-(Tyr-OMe)-PROXYL (398 mg, 68%) as a pale yellow powder.

**G1-Tyr-PROXYL.** Under dark conditions, G1-(Tyr-OMe)-PROXYL (300 mg, 52.2 µmol, 1 eq) and LiOH·H₂O (263 mg, 6.26 mmol, 120 eq) were dissolved in THF/H₂O 2:1 solution (20 mL) in a round-bottomed flask equipped with a stir bar. The reaction mixture was let to stir at room temperature during 24 hours. Afterwards, the solvent was evaporated to dryness to give a crude product that was purified by ultrafiltration (regenerated cellulose membrane 3kDa, 8 x 40 mL water). The concentrated was collected and the water eliminated by freeze-drying to afford G1-Tyr-PROXYL (252 mg, 85%) as a pale yellow solid.

**G2-Boc-Tyr-OMe.** In a Shlenk round-bottomed flask equipped with a stir bar under argon atmosphere, G2-Cl (200 mg, 41.8 mmol, 1 eq), Cs₂CO₃ (1.31 g, 4.01 mmol, 96 eq) and Boc-Tyr-OMe (592mg, 2.00 mmol, 48 eq) were suspended in anhydrous THF (20 mL). The reaction mixture was let under stirring 24 hours at room temperature. The solid residue was separated from the organic phase by filtration. The organic phase was collected and centrifuged to remove the remaining solid residue. Next, the solvent was evaporated and the resulting crude product was dissolved in a minimum volume of THF and precipitated several times with n-pentane. The precipitate was subjected to column chromatography using CH₂Cl₂:CH₃OH (15:1) as eluent, to afford G2-Boc-Tyr-OMe (430 mg, 63 %) as a pale yellow solid. ³¹P{¹H} NMR (100 MHz, CDCl₃) δ: 62.73, 62.56, 8.93; ¹H NMR (400 MHz, CDCl₃) δ: 7.75-7.52 (m, 54 H), 7.19 (d, *J* = 8.07 Hz, 24H), 7.14-6.88 (m, 108H), 5.05 (d, *J* = 7.28 Hz, 24H), 4.59-4.38 (m, 24H), 3.61 (s, 72H), 3.27 (d, *J* = 10.0 Hz, 54H), 3.10-2.84 (m, 48H), 1.37 (s, 216H).¹³C NMR (90.5 MHz, CDCl₃) δ: 172.1, 155.0, 151.5, 151.1, 149.6, 139.0, 138.5, 133.3, 132.4 132.1, 130.3, 128.3, 121.7, 121.4, 79.9, 54.4, 52.2, 37.6, 33.0, 32.9, 28.3.

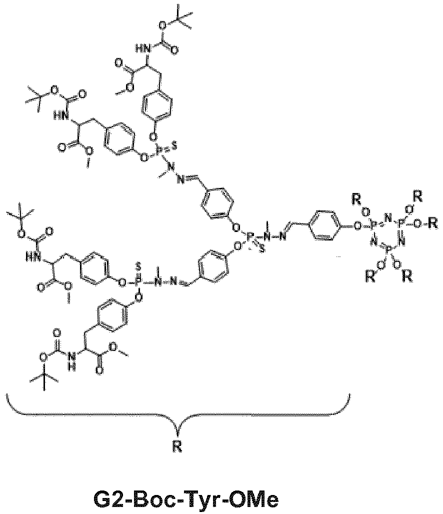

**G2-(Tyr-OMe)-PROXYL.** In a Shlenk round-bottomed flask equipped with a stir bar under argon atmosphere, G2-Boc-Tyr-OMe (200 mg, 18.2 µmol, 1 eq) was dissolved in anhydrous CH₂Cl₂ (5 mL). The solution was cooled in an ice bath and TFA (1.01 mL, 720 eq) was added dropwise during 20 min. The ice bath was removed, and the reaction mixture was stirred at room temperature for 5 h. Then, the solvent was evaporated to dryness and the crude was dissolved and evaporated several times with CH₃OH to remove TFA residue. Under dark conditions, the crude was transferred to a Shlenk round-bottomed flask equipped with a stir bar and dried under vacuum. In another Shlenk round-bottomed flask equipped with a stir bar and under argon atmosphere, 3-carboxy-PROXYL (121 mg, 652 µmol, 48 eq) and HATU (248 mg, 652 µmol, 48 eq) were dissolved in anhydrous CH₂Cl₂ (20 mL). Afterwards, DIPEA (568 µL, 3.26 mmol, 240 eq) was added. After being stirred for 20 minutes, this solution was transferred dropwise via cannula to the flask containing the crude. The reaction mixture was let to stir at room temperature during 24 hours. The solvent was evaporated and the crude product was dissolved in CH₂Cl₂ and washed with H₂O (4 x 50 mL). The organic phase was dried over MgSO₄ and concentrate under reduced pressure to give a crude product that was purified by ultrafiltration (regenerated cellulose membrane 5kDa, 7 x 40 mL acetone). The concentrated was collected and the solvent evaporated to afford G2-(Tyr-OMe)-PROXYL (120 mg, 70%) as a pale yellow powder.

**G2-Tyr-PROXYL.** Under dark conditions, G2-(Tyr-OMe)-PROXYL (100 mg, 7.91 µmol, 1 eq) and LiOH·H₂O (79.7 mg, 1.90 mmol, 240 eq) were dissolved in THF/H₂O 2:1 solution (6 ml) in round-bottomed flask equipped with a stir bar. The reaction mixture was let to stir at room temperature during 24 hours. Afterwards, the solvent was evaporated to dryness to give a crude product that was purified by ultrafiltration (regenerated cellulose membrane 5kDa, 8 x 40 mL water). The concentrated was collected and the water eliminated by freeze-drying to afford G2-Tyr-PROXYL (90 mg, 91%) as a pale yellow solid.

**G3-Boc-Tyr-OMe.** In a Shlenk round-bottomed flask equipped with a stir bar under argon atmosphere, P(S)Cl₃ terminated G3-CI (200 mg, 18.7 µmol, 1 eq), Cs₂CO₃ (1.46 g, 4.48 mmol, 240 eq) and Boc-Tyr-OMe (582mg, 1.97 mmol, 105 eq) were added and suspended in anhydrous THF (20 mL).The reaction mixture was let under vigorous stirring during 44 hours at room temperature. The solid residue was separated from the organic phase by filtration. The organic phase was collected and centrifuged to remove the remaining solid residue. Next, the solvent was evaporated and the resulting crude product was dissolved in a minimum volume of THF and precipitated several times with n-pentane. The precipitate was dried under vacuum and dissolved again in acetone to be purified by ultrafiltration (regenerated cellulose membrane 3 kDa, 7 x 40 mL acetone) to afford G3-Boc-Tyr-OMe (380 mg, 88 %) as a pale yellow solid. ³¹P{¹H} NMR (100 MHz, CDCl₃) δ: 63.32, 8.93; ¹H NMR (360 MHz, CDCl₃) δ: 7.78-7.47 (m, 126H), 7.25-6.93 (m, 276H), 5.05 (br s, 48H), 4.50 (br s, 48H), 3.61 (br s, 144H), 3.27 (m, 120H), 2.98 (m, 96H), 1.36 (s, 432H); ¹³C NMR (90.5 MHz, CDCl₃) δ: 172.3, 155.1, 151.4, 151.3, 149.7, 149.67, 139.2, 132.8, 138.7, 133.4, 132.5, 128.4, 121.9, 121.5, 80.0, 54.5, 52.3, 37.8, 33.1, 33.0, 28.4.

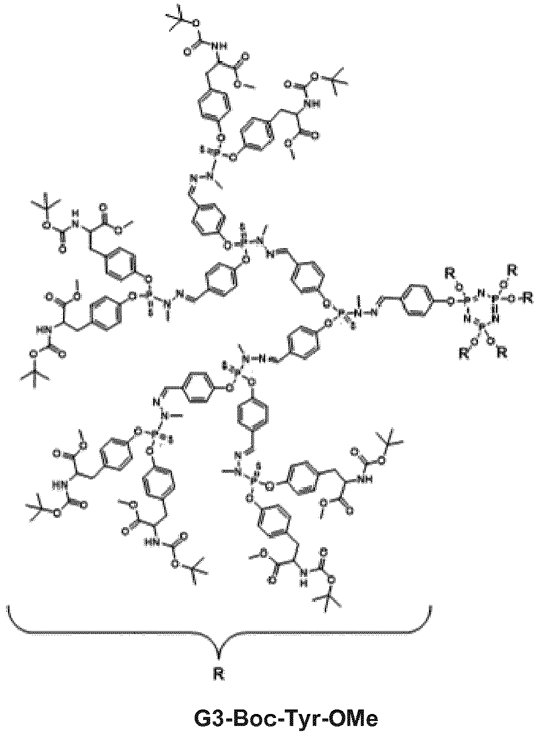

**G3-(Tyr-OMe)-PROXYL.** In a Shlenk round-bottomed flask equipped with a stir bar under argon atmosphere, G3-Boc-Tyr-OMe (300 mg, 13.0 µmol, 1 eq) was dissolved in anhydrous CH₂Cl₂ (25 mL). The solution was cooled in an ice bath and TFA (1.44 mL, 1440 eq) was added dropwise. The ice bath was removed, and the reaction mixture was stirred at room temperature for 5 h. Then, the solvent was evaporated to dryness and the crude was dissolved and evaporated several times with CH₃OH to remove TFA residue. Next, the crude was transferred to a Shlenk round-bottomed flask equipped with a stir bar and dried under vacuum. In another Shlenk round-bottomed flask equipped with a stir bar and under argon atmosphere, 3-carboxy-PROXYL (290 mg, 1.56 mmol, 120 eq) and HATU (592 mg, 1.56 mmol, 120 eq) were dissolved in anhydrous CH₂Cl₂ (25 mL). Afterwards, DIPEA (1.09 mL, 6.23 mmol, 480 eq) was added. After being stirred for 20 minutes, this solution was transferred dropwise via cannula to the flask containing the crude. The reaction mixture was let to stir at room temperature during 40 hours. The solvent was evaporated and the crude product was dissolved in CH₂Cl₂ and washed with H₂O (4 x 50 mL). The organic phase was dried over MgSO₄ and concentrate under reduced pressure to give a crude product that was purified by ultrafiltration (regenerated cellulose membrane 10 kDa, 6 x 40 mL acetone followed by 30 kDa 3 x 40 mL acetone). The concentrated was collected and the solvent evaporated to afford G3-(Tyr-OMe)-PROXYL (238 mg, 69%) as a pale yellow powder.

**G3-Tyr-PROXYL.** Under dark conditions, G3-(Tyr-OMe)-PROXYL (200 mg, 7.58 µmol, 1 eq) and LiOH·H₂O (153 mg, 3.64 mmol, 480 eq) were dissolved in THF/H₂O 2:1 solution (16 ml) in round-bottomed flask equipped with a stir bar. The reaction mixture was let to stir at room temperature during 24 hours. Afterwards, the solvent was evaporated to dryness to give a crude product that was purified by ultrafiltration (regenerated cellulose membrane 5kDa, 8 x 40 mL water). The concentrated was collected and the water eliminated by freeze-drying to afford G3-Tyr-PROXYL (181 mg, 92%) as a pale yellow solid.

### RESULTS AND DISCUSION

### EPR and DLS characterization.

Considering our goal to obtain dendrimers fully functionalized with nitroxyl radicals, it was crucial to quantify the functionalization by EPR since the presence of radicals make impossible a quantitative evaluation by NMR. The EPR spectra of 0.21 mM solutions of 3-carboxy-PROXYL and Gn-(Tyr-OMe)-PROXYL dendrimers (CH₂Cl₂, 300 K) are presented in Figure 2.

The spectrum of free 3-carboxy-PROXYL presents a three resolved sharp lines centered at g = 2.0062, with similar intensities and hyperfine constant (a_{N} ≈ 14.5 G), due to the coupling between the unpaired electron and the ¹⁴N nucleus. In contrast, the EPR spectra of Gn-(Tyr-OMe)-PROXYL dendrimers are progressively dominated by a broad line signal, resulting from the spin exchange and dipole-dipole interactions between PROXYL radical units. The increasing lack of spectral resolution is consistent with the increasing number of nitroxyl end groups that are interacting with each other, with several hyperfine transitions (decrease of the splitting value a_{N}/*n* and an increase of the number of lines 2*n*+1, where *n* is the number of interacting radicals).

The calculated double integral of all spectra in Figure 2, carried out at the same concentration and EPR's acquisition parameters, reflects the increase in the radical number with the increase of generation. This correlation resulted in a good linearity with the theoretical number of pendant PROXYL units. The integrals increase nearly the double with the increase of generation and the integral of G0 is nearly six times higher than that of 3-carboxy-PROXYL. Considering that the small deviations in the calculated integrals are explainable by the common sources of error in quantitative EPR, it can be contemplated a practical full functionalization of the dendrimers for all generations, at this synthetic step.

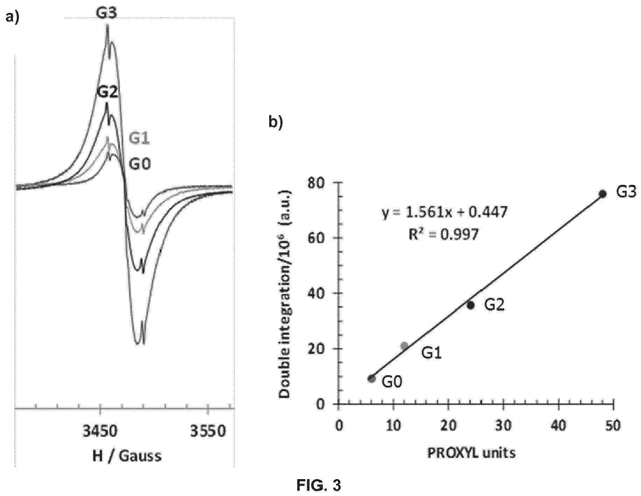

The next stage was the hydrolysis of the methyl ester group of Gn-(Tyr-OMe)-PROXYL dendrimers to obtain the water-soluble Gn-Tyr-PROXYL dendrimers. The EPR spectra of 0.21 mM solutions of Gn-Tyr-PROXYL dendrimers in 100 mM phosphate buffer (PB) pH 7.4 at 300 K are presented in Figure 3. For all generations, the EPR spectra are dominated by a broad and single intense line.

The determined double integral of the EPR spectra was calculated from the experiment at 180 K (Figure 3b) due to the higher signal-to-noise ratio (no interference of the dielectric absorption of water). The good correlation observed between the number of radicals and the double integral corroborates the full functionalization of PROXYL radical units. Thus, the hydrolysis step did not affect the content of nitroxyl radicals at the periphery.

All generations of both families of radical dendrimers, Gn-(Tyr-OMe)-PROXYL and Gn-Tyr-PROXYL, presented a |Δ*m*ₛ| = 2 transition at half-field in frozen conditions. This signal is characteristic of dipolar coupled spins and gives a direct evidence of the presence of a high-spin state. As it was observed in diluted conditions, its origin is due to intramolecular dipolar interactions, as expected for the proximity of the radical units. The intensity of such a signal increases generation by generation by a factor of 2, observing a perfectly direct proportionality between the |Δ*m*ₛ| = 2 signal intensity and the number of radicals of each generation.

The average hydrodynamic diameter of Gn-Tyr-PROXYL dendrimers, determined by DLS in similar conditions (Table 2) ranged from 2.8 nm for G0-, to 4.8 nm to G3-Tyr-PROXYL, being similar to other water-soluble dendrimers in the same range of molecular weight. It is worth saying that none of the Gn-Tyr-PROXYL dendrimer generations showed significant aggregation at physiological pH (< 0.2 % by volume).

**Table 1. Hydrodynamic diameter of Gn-Tyr-PROXYL dendrimers (100 mM phosphate buffer (PB) pH 7.4 at 300 K) shown in the Z-average with standard deviation values (N = 5).**

| Compound | Z-av size DLS (nm) PB |
|---|---|
| G0-Tyr-PROXYL | **2.81 ±0.58** |
| G1-Tyr-PROXYL | **3.44 ±0.75** |
| G2-Tyr-PROXYL | **4.23 ±1.30** |
| G3-Tyr-PROXYL | **4.78 ±1.23** |

### Rate of PROXYL reduction.

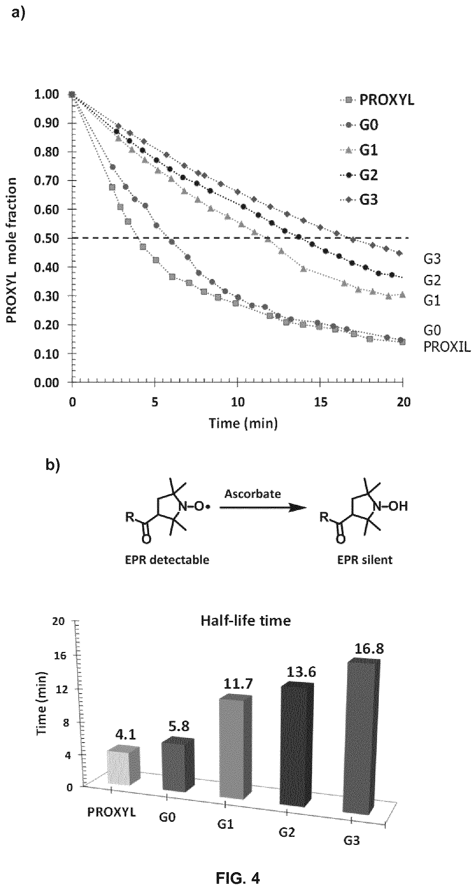

The effect of the dendritic scaffold on nitroxyl rate of reduction was investigated under pseudo-first-order conditions, using a 10-fold excess of sodium ascorbate per PROXYL unit in 100 mM PB pH 7.4 at 300 K. The reduction was monitored by EPR spectroscopy following the decrease of the area under the spectra as a function of time (Figure 4a). As reference, the rate of reduction of 3-carboxy-PROXYL was determined under identical conditions. The reduction of PROXYL groups did not show simple first-order kinetics. It was possible to identify different stages of reduction, with different kinetics. The first stage, where the reduction of more than 50% of the available PROXYL units (half-life) happened (Table 2), presented a faster kinetics. As observed in Figure 4b, the half-life time of PROXYL units increased with the increase in generation number, being in the case of G3-Tyr-PROXYL, four times higher than that of free 3-carboxy-PROXYL radical. This increase of resistance against chemical reduction could be explained by conformation changes with the increase of generation. It is known that dendrimers progressively change from an open to a more globular architecture, due to the possibility of back-folding of the terminal groups. As a result, peripheral nitroxyl groups are less easily accessible by the ascorbate ions, making their chemical reduction by ascorbate ions more difficult.

**Table 2: Rates of nitroxide reduction with sodium ascorbate (10-fold excess per PROXYL unit) for 3-carboxy-PROXYL and Gn-Tyr-PROXYL dendrimers. Rate constants were determined by EPR spectroscopy following the decrease of the area under the spectra as a function of time.**

| Compound | Initial stage k (M⁻¹s⁻¹) x 10³ | *R*₂ |
|---|---|---|
| 3-carboxy PROXYL | **11.2** | **0.992** |
| GO-Tyr-PROXYL | **7.88** | **0.990** |
| G1-Tyr-PROXYL | **3.91** | **0.995** |
| G2-Tyr-PROXYL | **3.35** | **0.997** |
| G3-Tyr-PROXYL | **2.74** | **0.998** |

### Relaxivity measurements.

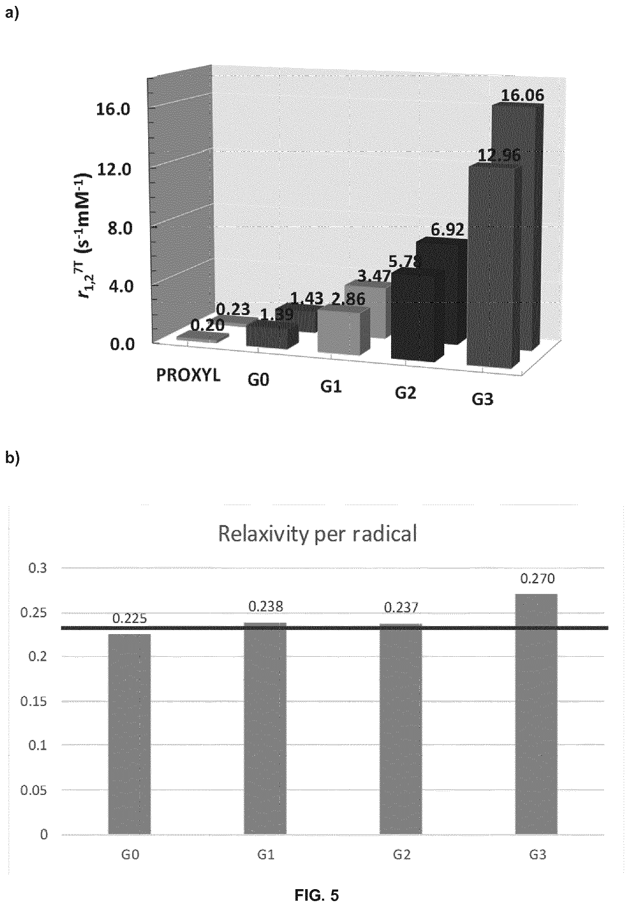

The *r*₁ and *r*₂ relaxivity constants per unit of nitroxyl radical where obtained at 7 T in 100 mM phosphate buffer pH 7.4 and 300K (Table 3). The plots of proton relaxation rates ¹H *R*₁ and ¹H *R*₂ (*R*ₙ = 1/*T*ₙ) of water molecules versus the nitroxyl units' concentration were linear for all the agents, over the concentration range studied (0.5-10 mM). The presence of a high number of paramagnetic nitroxides in the dendrimer's periphery, as expected, resulted in high molecular relaxivities (Figure 5a). While G1-Tyr-PROXYL dendrimer presents a similar relaxivity (2.9 s⁻¹mM⁻¹) than the most widely used GdCA in clinics Magnevist (Gd-DTPA, 3.2 s⁻¹mM⁻¹ r.t. 7T), one molecule of G3-Tyr-PROXYL dendrimer presents a relaxivity of ca. 13 s⁻¹mM⁻¹ that means a remarkably 4 times higher value than Gd-DTPA. In addition, G3-Tyr-PROXYL dendrimer presents a 16% higher relaxivity per radical than the relaxivity per radical of G0-G2 dendriders (Figure 5b).

Furthermore, the control over the size of the dendrimers, opens the opportunity to modulate their distribution profile in the body, which is impossible in the case of Gd-DTPA. On the contrary to the works reported previously based on PPI and PAMAM nitroxyl terminated dendrimers, the inventors did not observe a decrease in *r*₁ and *r*₂ up to G3 (48 terminal nitroxyl groups). This improvement in relaxivity most probably is due to the higher solubility of the negatively charged Gn-Tyr-PROXYL dendrimers at physiological pH. In all generations, the ratio *r*₁/*r*₂ was near 1, what makes favorable their use as *T*₁ imaging CA, but clearly the dendrimer of third generation is the best candidate for use as a contrast agent.

**Table 3. Longitudinal r₁ and transverse r₂ relaxivity constants per unit of nitroxyl radical determined at 7T in 100 mM phosphate buffer pH 7.4, 300K.**

| Compound | r₁^{7T} (mM⁻¹s⁻¹) | *r*₂^{7T} (mM⁻¹s⁻¹) |
|---|---|---|
| 3-carboxy PROXYL | **0.20**±0.014 | **0.23**±0.06 |
| GO-Tyr-PROXYL | **0.23**±0.014 | **0.24**±0.08 |
| G1-Tyr-PROXYL | **0.24**±0.007 | 0.29±0.05 |
| G2-Tyr-PROXYL | **0.24**±0.008 | 0.29±0.03 |
| G3-Tyr-PROXYL | **0.27**±0.009 | 0.33±0.09 |

### Cytotoxicity.

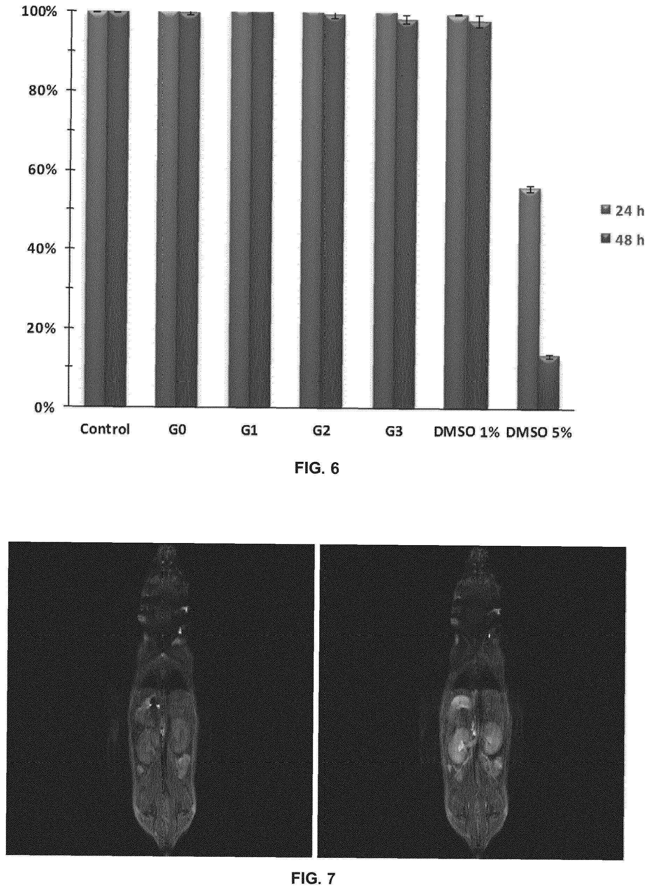

To assess the toxicity of Gn-Tyr-PROXYL dendrimers, *in vitro* viability assays were conducted with a fetus normal lung tissue cell line (MRC-5). In these assays, the cells were incubated with Gn-Tyr-PROXYL dendrimers (n=0-3) of different concentrations ranging from 0.016 to 2mM per radical (Figure 6) for 24 and 48 h. Cell viability was determined by the MTT assay. As a result, Gn-Tyr-PROXYL dendrimers (n=0-3) did not show any cytotoxicity *in vitro,* in the tested concentration range (see Figure 6).

It is known that nitroxides on their own are not considered cytotoxic and negatively charged dendrimers do not interact with biological environment, since they are repelled by the negatively charged cell membrane. The combination of these two attributes is confirmed by the results, giving biocompatible dendrimers better suited for clinical applications.

## Claims

1. Compound of formula (I): 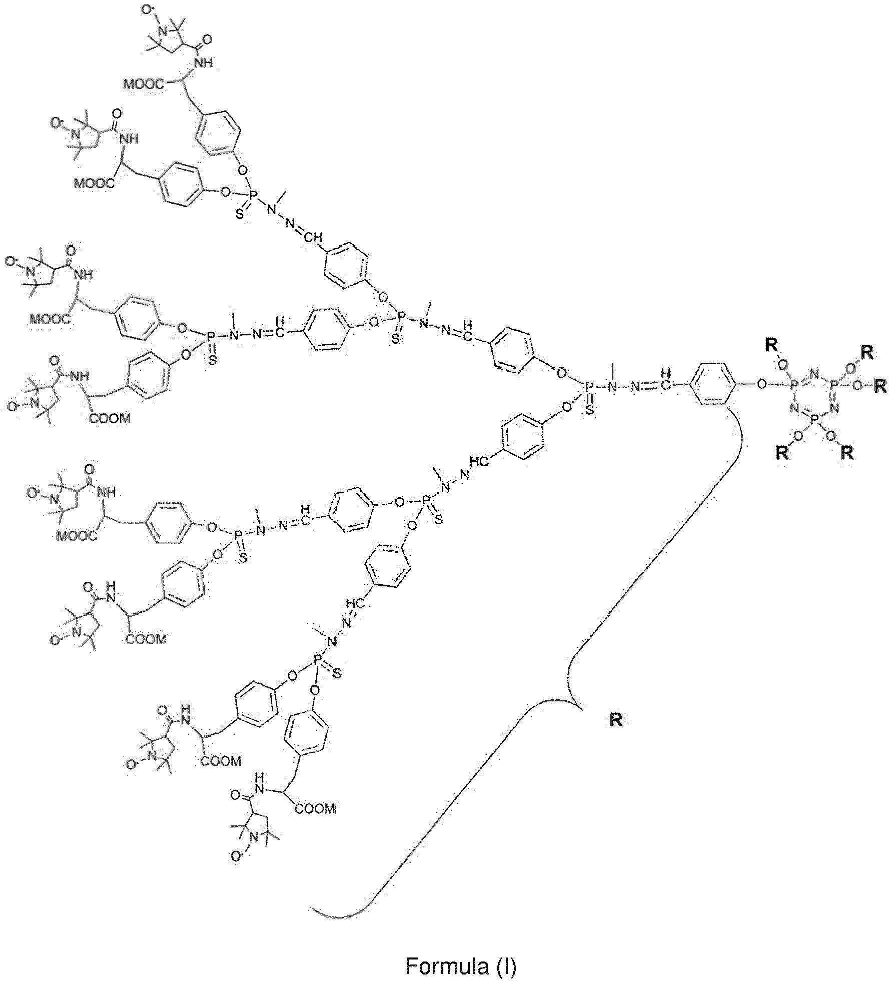 its tautomers and/or solvates, wherein M is a cation selected from Li+, Na⁺ or K⁺,

2. Compound of formula (I) according to claim 1, wherein M is Li+.

3. Pharmaceutical composition comprising the compound of formula (I) as described in claim 1 or 2.

4. Pharmaceutical composition according to claim 3 which is an injectable composition.

5. Compound of formula (II): 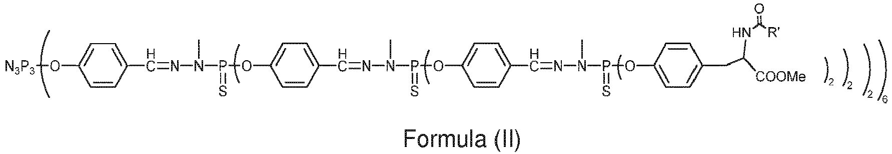 wherein R' is: 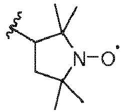 or -O-CH (CH₃)₃ its tautomers and/or solvates.

6. A process of obtainment of the compound of formula (I) described in claim 1 or 2, from the compound G3-CI: 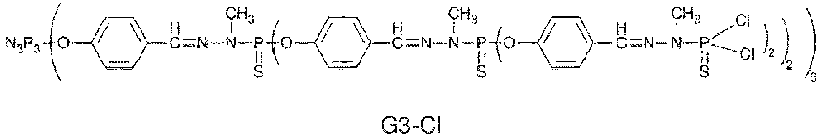 wherein the process comprises at least the following steps:
a) terminal chlorine groups attached to the phosphorus atoms of the compound G3-Cl are replaced by units of *N*-Boc-protected tyrosine methyl ester (Boc-Tyr-OMe), via phenolic groups, affording G3-Boc-Tyr-OMe dendrimer: 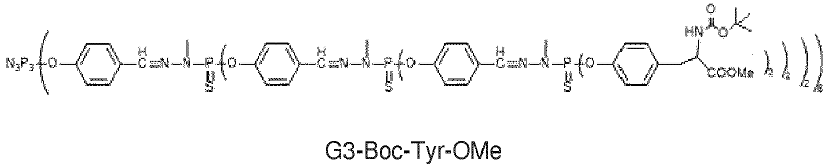
b) Subsequently, the amine groups of tyrosine are deprotected with trifluoroacetic acid and coupled with 3-carboxy-PROXYL radicals using HATU as coupling reagent and DIPEA as base to obtain the G3-(Tyr-OMe)-PROXYL dendrimer: 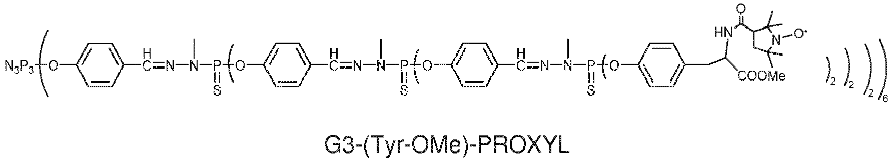
c) the methyl esters are hydrolyzed with LiOH, KOH or NaOH, resulting in the water soluble compound of formula (I).

7. The process according to claim 6, wherein the dendrimer obtained in step a) is purified by ultrafiltration.

8. The process according to claim 6, wherein the dendrimer obtained in step b) is purified by column chromatography and ultrafiltration.

9. Use of the compound of formula (I) described in claim 1 or 2, as a magnetic resonance imaging (MRI) contrast agent.
